# FASCICULE DE BREVET EUROPEEN

(11) **EP 0 607 080 B1**
(45) Date de publication et mention de la délivrance du brevet: **20.06.2001**
(21) Numéro de dépôt: 94400062.9
(22) Date de dépôt: 11.01.1994
(51) Int. Cl.: C12N 15/81, C12N 15/62, C12N 15/15, C12N 15/20, C12N 15/12, C12N 1/15, C07K 14/00

(54) **Nouvelle séquence pro permettant la sécrétion de protéines hétérologues à partir d'une cellule de levure**
Neue Prosequenz zur Sekretion heterologer Proteine aus Hefezellen
New pro sequence for secretion of heterologous proteins from yeast cells

(30) Priorité: 11.01.1993 FR 9300171
(43) Date de publication de la demande: 20.07.1994
(73) Titulaire: TRANSGENE S.A., 67082 Strasbourg Cédex (FR)
(72) Inventeur: Achstetter, Tilman, D-00760 Oberkirch (DE)
(74) Mandataire: Warcoin, Jacques

(56) Documents cités:
- EP-A- 0 261 534
- EP-A- 0 349 451
- WO-A-90/13646
- PROCEEDINGS OF THE NATIONAL ACADEMY OF SCIENCES OF USA. vol. 81 , Aoüt 1984 , WASHINGTON US pages 4642 - 4646 A. BRAKE ET AL 'alpha-Factor-directed synthesis and secretion of mature foreign proteins in Saccharomyces cerevisiea'
- INVERTEBRATE REPRODUCTION AND DEVELOPMENT vol. 21, no. 1 , 1992 pages 15 - 24 J-M REICHHART ET AL 'Expression and secretion in yeast of active insect defensin, an inducible antibacterial peptide from the fleshfly Phormia terranovae'
- GENE. vol. 77 , 1989 , AMSTERDAM NL pages 341 - 348 M. CHARLIER ET AL 'Cloning and expression of cDNA encoding ovine trophoblastin: its identity with a class-II alpha interferon'
- JOURNAL OF BIOLOGICAL CHEMISTRY. vol. 268, no. 22 , 5 Aoüt 1993 , BALTIMORE US pages 16458 - 16464 H. KOLBE ET AL 'Xenoxins, a family of peptides from dorsal gland secretion of Xenopus laevis related to snake venom cytotoxins and neurotoxins'
- EMBO JOURNAL. vol. 9, no. 8 , 1990 , EYNSHAM, OXFORD GB pages 2507 - 2515 J-L DIMARCQ ET AL 'Insect immunity: expression of the two major inducible antibacterial peptides, defensin and diptericin, in Phormia terranovae'
- S. CAPLAN ET AL.: "", JOURNAL OF BACTERIOLOGY, USA, Janvier 1991, Vol. 173, no. 2, pages 627 à 635
- A.J. BRAKE: "", METHODS IN ENZYMOLOGY, , 1990, Vol. 185, no. , pages 408 à

## Description

La présente invention a trait à une nouvelle séquence pro capable de promouvoir la maturation de précurseurs de protéines hétérologues et la sécrétion desdites protéines hétérologues, à partir d'une cellule de levure recombinante. La nouvelle séquence pro est dérivée du précurseur de la défensine A d'insecte. L'invention concerne plus particulièrement une cassette d'expression, un vecteur ainsi qu'une cellule de levure contenant un fragment d'ADN codant pour ladite séquence pro et un procédé de préparation de protéines hétérologues mettant en oeuvre une telle séquence. La présente invention est particulièrement destinée à la production par la levure, notamment une levure du genre *Saccharomyces cerevisiae* (*S*. *cerevisiae*), de protéines hétérologues présentant un intérêt industriel, qui seront secrétées dans le milieu de culture des levures dans lequel elles pourront être récupérées.

Les levures sont des organismes eucaryotes unicellulaires. Depuis déjà de nombreuses années, les levures et plus particulièrement les levures du genre *S*. *cerevisiae*, sont couramment utilisées pour la production de protéines d'intérêt industriel. En effet, la génétique et la biochimie de cette souche ont été étudiées intensivement en laboratoire.

Leur intérêt à titre de cellules hôte repose sur le fait qu'il s'agit d'organismes se multipliant rapidement dans des milieux non coûteux et dont la génétique peut être facilement manipulée en utilisant les techniques de l'ADN recombinant.

On a établi que la levure est capable de secréter quelques protéines dans le milieu externe. D'une manière générale, le processus de sécrétion est un processus complexe, qui nécessite le passage de la protéine à secréter, du cytoplasme dans le réticulum endoplasmique pour, en bout de course, être secrétée dans le milieu externe.

D'une manière générale, les protéines secrétables sont exprimées sous la forme d'un précurseur contenant une séquence signal amino-terminale. De nombreux précurseurs de protéines secrétées et de protéines membranaires ont été décrits dans la littérature, tant chez les procaryotes que chez les eucaryotes.

D'une manière générale, une séquence signal contient un grand nombre de résidus d'acides aminés hydrophobes. Sa fonction est de promouvoir le passage d'un précurseur d'une protéine devant être secrétée, dans le réticulum endoplasmique. Au cours de cette étape de la sécrétion, la séquence signal est clivée par une endopeptidase associée à la membrane, encore appelée signal peptidase, pour donner la protéine mature.

Ainsi, pour qu'une protéine hétérologue soit secrétée dans la levure, elle doit être précédée d'une séquence signal, encore appelée séquence pré, reconnue par la levure. En général, on utilise une séquence signal homologue, issue d'une protéine secrétable de la levure, qui va diriger la sécrétion de la protéine de fusion, séquence signal homologue-protéine hétérologue mature.

En outre, la sécrétion peut nécessiter la présence additionnelle d'une séquence dans le précurseur de la protéine destinée à être secrétée, cette séquence supplémentaire étant appelée séquence pro. Ainsi, la protéine à secréter est exprimée sous la forme d'un précurseur pré-pro-protéine. De nombreuses protéines de levure naturellement secrétées contiennent, outre la séquence pré, une séquence pro.

Ainsi, le facteurα (MFα) qui dérive du gène MFα1, est une phéromone sexuelle secrétée par les cellules haploïdes de *S*. *cerevisiae* de type MATα. Le facteur MFα1 est initialement synthétisé sous la forme d'un précurseur pré-pro-facteurα. Ledit précurseur consiste en une séquence signal ou séquence pré de 19 acides aminés, suivie d'une séquence pro de 64 acides aminés contenant trois sites de glycosylation, elle même suivie d'une séquence Lys-Arg précédant 4 copies du facteur α séparées par des peptides espaceurs contenant entre autre le dipeptide Lys-Arg (Kurjan et Herskowitz, Cell, 1982, *30*, 933-943).

Il est connu que le système endogène de sécrétion de la levure peut être mis en oeuvre pour permettre la sécrétion de protéines hétérologues matures dans le milieu de culture. C'est un système particulièrement avantageux d'un point de vue industriel, puisque la protéine hétérologue peut être récupérée directement du milieu de culture, évitant ainsi les étapes de broyage ou de cassage des cellules difficiles à maîtriser et éliminant les risques de dégradation intracellulaire dus aux nombreuses protéases contenues dans une levure.

Ainsi, de nombreuses publications relatent l'utilisation des séquences pré et pro du facteurα codées par le gène MFα1 (pré MFα1, pro MFα1), pour produire un grand nombre de protéines étrangères à la levure, par exemple l'hirudine. L'hirudine est un inhibiteur très spécifique et efficace de la thrombine. Il s'agit donc d'un agent thérapeutique très intéressant présentant une activité anticoagulante. Un certain nombre de variants de l'hirudine ont été identifiés et produits dans des souches de *S*. *cerevisiae*, en utilisant le procédé de sécrétion mettant en oeuvre les séquences pré et pro MFα1, comme cela est décrit dans les demandes EPA 0252854 et EPA 0273800 au nom de la demanderesse.

D'autre part, Dimarcq et al. (EMBO J., 1990, 9, 2507-2515) ont récemment mis en évidence que les larves d'insecte *Phormia terranovae,* dans lesquelles ont été injectées des bactéries, produisent et secrètent dans le sang un produit bactéricide anti gram+, encore appelé défensine A. Ces auteurs ont publié la séquence d'un ADN complémentaire (ADNc) codant pour un précurseur de la défensine A. Le précurseur est constitué de 94 acides aminés, dont les 40 acides aminés C-terminaux correspondent à la défensine A mature. La séquence pré comprend les 23 ou 28 premiers acides aminés N-terminaux et montre toutes les caractéristiques d'une séquence signal (nature hydrophobe et présence d'un site potentiel de clivage par une signal peptidase après l'alanine en position 23 ou l'alanine en position 28). La séquence pré est suivie d'une séquence pro de 31 ou 26 acides aminés, selon la longueur de la séquence pré, qui se termine par un dipeptide basique Lys-Arg.

Enfin, Reichhart et al ( Invertebrate Reproduction and Development, 1892, 21:1, 15-24, mentionne que la levure est capable d'exprimer et de sécréter la défensine A d'insecte).

On a maintenant trouvé que la séquence pro du précurseur de la défensine A peut être utilisée pour secréter efficacement des protéines hétérologues dans la levure, notamment dans la levure du genre *S*. *cerevisiae*.

L'utilisation de ladite séquence pro de défensine A est notamment utile pour produire dans la levure des protéines hétérologues présentant un intérêt industriel, comme par exemple l'hirudine ou la trophoblastine d'ovin contenant, en outre, une extension N-terminale constituée par le dipeptide Ala-Pro, ladite extension n'ayant aucun effet sur l'activité biologique du produit (Degryse et al., Gene, 1992, *118,* 47-53).

La présente invention a donc pour objet une cassette d'expression permettant la synthèse d'une protéine hétérologue mature, à l'exclusion de la défensine de Phormia terranovae, comprenant au moins un fragment d'ADN codant pour un précurseur de ladite protéine hétérologue ; ledit précurseur comprenant :
- une séquence pré fonctionnelle dans la levure,
- la séquence pro du précurseur de la défensine A de *Phormia terranovae*, un variant ou un fragment fonctionnel de celle-ci, et
- une séquence correspondant à celle de ladite protéine hétérologue sous forme mature ;
ledit fragment d'ADN étant sous le contrôle des éléments nécessaires à son expression.

Par protéine hétérologue, on entend toute protéine qui n'est pas produite naturellement par la levure, ainsi que toute protéine qui n'est pas produite naturellement par *Phormia terranovae*.

Les protéines hétérologues matures qui peuvent être synthétisées dans un tel système peuvent être constituées par toute protéine hétérologue mature native. Cependant d'une manière alternative, une protéine hétérologue mature peut être constituée par la fusion de deux ou plusieurs polypeptides d'intérêt, d'origine diverse ou par une protéine hétérologue mutée par rapport à la protéine hétérologue native, de manière à présenter une activité biologique améliorée ou modifiée.

A titre d'exemple, on cite l'hirudine, notamment son variant rHV2-Lys⁴⁷, la trophoblastine ovine comprenant une extension N-terminale constituée par le dipeptide Ala-Pro ainsi que les xénoxines, famille de peptides isolés de *Xenopus laevis* (Kolbe et al., J. of Biol. Chem., 1993, *268*, 16458-16464).

Par séquence pré, on entend une séquence pré principalement hydrophobe en nature, présente à l'extrémité N-terminale de la forme précurseur d'une protéine destinée à être secrétée et dont la fonction est de permettre au précurseur de ladite protéine d'entrer dans le chemin de secrétion, en particulier dans le réticulum endoplasmique, d'une cellule de levure. La séquence pré est normalement clivée au cours du processus de sécrétion par une signal peptidase endogène.

La séquence pré peut être naturelle ou synthétique, hétérologue ou homologue à la levure, peut être constituée par un variant d'une séquence pré naturelle ou par un fragment fonctionnel de celle-ci. Par fragment fonctionnel, on entend tout fragment d'une séquence pré naturelle capable d'assurer l'entrée d'un précurseur d'une protéine secrétable dans le chemin de secrétion d'une cellule de levure. De préférence, on utilisera un fragment d'ADN codant pour un précurseur dont la séquence pré est une séquence pré homologue à la levure, par exemple la séquence pré du précurseur du facteur α (MFα1), de l'invertase ou de la β-glucanase 2 (BGL2) de *S*. *cerevisiae.*

Une séquence pro du précurseur codé par le fragment d'ADN utilisé dans la cassette d'expression selon l'invention, peut avoir notamment une séquence telle que montrée dans l'identificateur de séquence SEQ ID NO:1, débutant à l'acide aminé en position +1 ou +6 et se terminant à l'acide aminé +31, dans laquelle, ensemble ou séparément, le résidu Xaa en position +2 est un résidu proline ou cystéine, le résidu Xaa en position +6 est un résidu alanine ou cystéine, le résidu Xaa en position +9 est un résidu alanine cystéine ou thréonine, le résidu Xaa en position +17 est un résidu alanine ou cystéine, le résidu Xaa en position +28 est un résidu arginine ou proline et le résidu Xaa en position +30 est un résidu arginine ou lysine.

On préfère notamment mettre en oeuvre la séquence pro native du précurseur de la défensine A de *Phormia terranovae*, telle que montrée dans l'identificateur de séquence SEQ ID NO: 2.

Un variant de la séquence pro, peut comporter plusieurs mutations par rapport à la séquence pro native du précurseur de la défensine A de *Phormia terranovae*, notamment la substitution, l'addition ou la délétion d'un ou plusieurs acides aminés, à la condition qu'un tel variant soit capable de diriger un précurseur d'une protéine hétérologue secrétable du réticulum endoplasmique à l'appareil de Golgi puis de permettre la secrétion de ladite protéine hétérologue dans le milieu de culture d'une cellule de levure. On cite plus particulièrement les variants comprenant un résidu cystéine aux positions +2, +6, +9 ou +17 et/ou un résidu proline en position +29.

De manière plus particulière, une séquence pro a notamment une séquence dont le degré d'homologie avec la séquence pro native du précurseur de la défensine A de *Phormia terranovae* est supérieur à 80%, de manière avantageuse supérieur à 90% et de préférence supérieur à 95%.

Au cours du processus de sécrétion, la séquence pro est clivée par une endoprotéase, laquelle reconnait à l'extrémité C-terminale de ladite séquence pro, une séquence cible constituée par un dipeptide basique de type Lys-Arg ou Arg-Arg et clive après ladite séquence cible, libérant ainsi la protéine hétérologue mature. L'endoprotéase peut être homologue ou hérérologue à la levure, peut être constituée par un variant ou un fragment fonctionnel de celle-ci. Par endoprotéase, on entend toute endoprotéase capable de reconnaître et cliver après une séquence cible située à l'extrémité C-terminale de ladite séquence pro, notamment après un dipeptide basique Lys-Arg ou Arg-Arg. On peut citer, par exemple, le produit de tout ou partie du gène KEX2 de *S*. *cerevisiae* ou une endoprotéase hétérologue exprimée dans la cellule de levure, comme la furine des cellules de mammifères.

Ainsi la séquence pro comporte à son extrémité C-terminale un dipeptide basique Lys-Arg ou Arg-Arg permettant de cliver la séquence pro de la protéine à secréter probablement dans l'appareil de Golgi, libérant ainsi la forme mature de ladite protéine.

Selon un mode préféré, la séquence pro a la séquence en acides aminés telle que montrée dans l'identificateur de séquence SEQ ID NO:1, débutant à l'acide aminé en position +1 ou +6 et se terminant à l'acide aminé +31, dans laquelle le résidu Xaa en position +2 est un résidu proline, le résidu Xaa en position +6 est un résidu alanine, le résidu Xaa en position +9 est un résidu alanine ou thréonine, le résidu Xaa en position +17 est un résidu alanine, le résidu Xaa en position +28 est un résidu arginine et le résidu Xaa en position +30 est un résidu arginine ou lysine.

Ainsi, la séquence pro est avantageusement choisie parmi :
(1) la séquence pro du précurseur de la défensine A de *Phormia terranovae*, débutant à l'acide aminé en position +1 et se terminant à l'acide aminé +31, dans laquelle le résidu Xaa en position +9 est un résidu alanine et le résidu Xaa en position +30 est un résidu arginine.
(2) la séquence pro du précurseur de la défensine A de *Phormia terranovae*, débutant à l'acide aminé en position +1 et se terminant à l'acide aminé +31, dans laquelle le résidu Xaa en position +9 est un résidu thréonine et le résidu Xaa en position +30 est un résidu arginine.
(3) la séquence pro du précurseur de la défensine A de *Phormia terranovae*, débutant à l'acide aminé en position +1 et se terminant à l'acide aminé +31, dans laquelle le résidu Xaa en position +9 est un résidu alanine et le résidu Xaa en position +30 est un résidu lysine.
(4) la séquence pro du précurseur de la défensine A de *Phormia terranovae*, débutant à l'acide aminé en position +1 et se terminant à l'acide aminé +31, dans laquelle le résidu Xaa en position +9 est un résidu thréonine et le résidu Xaa en position +30 est un résidu lysine.
(5) la séquence pro du précurseur de la défensine A de *Phormia terranovae*, débutant à l'acide aminé en position +6 et se terminant à l'acide aminé +31, dans laquelle le résidu Xaa en position +9 est un résidu alanine et le résidu Xaa en position +30 est un résidu arginine.
(6) la séquence pro du précurseur de la défensine A de *Phormia terranovae*, débutant à l'acide aminé en position +6 et se terminant à l'acide aminé +31, dans laquelle le résidu Xaa en position +9 est un résidu thréonine et le résidu Xaa en position +30 est un résidu arginine.
(7) la séquence pro du précurseur de la défensine A de *Phormia terranovae*, débutant à l'acide aminé en position +6 et se terminant à l'acide aminé +31, dans laquelle le résidu Xaa en position +9 est un résidu alanine et le résidu Xaa en position +30 est un résidu lysine.
(8) la séquence pro du précurseur de la défensine A de *Phormia terranovae*, débutant à l'acide aminé en position +6 et se terminant à l'acide aminé +31, dans laquelle le résidu Xaa en position +9 est un résidu thréonine et le résidu Xaa en position +30 est un résidu lysine.

Par éléments nécessaires à l'expression du fragment d'ADN codant pour un précurseur d'une protéine hétérologue, on entend l'ensemble des éléments permettant la transcription dudit fragment d'ADN en ARN messager (ARNm) et la traduction de l'ARNm en protéine.

La cassette d'expression selon l'invention, comporte notamment un promoteur fonctionnel dans une cellule de levure. De tels promoteurs sont bien connus de l'homme de l'art et sont insérés dans la cassette d'expression en amont du fragment d'ADN à transcrire, par les techniques conventionnelles du génie génétique. On citera, par exemple, les promoteurs du gène PGK (phosphoglycérate kinase), du gène MFβ1 et du gène ADH (alcool-dehydrogénase) de levure.

Une cassette d'expression selon l'invention, présentant un intérêt particulier, est celle qui associe un fragment d'ADN codant pour un précurseur de l'hirudine, comportant la séquence pré du précurseur du facteurα de la levure, la séquence pro du précurseur de la défensine A de *Phormia terranovae*, débutant à l'acide aminé en position +1 et se terminant à l'acide aminé +31, dans laquelle le résidu Xaa en position +9 est un résidu alanine et le résidu Xaa en position +30 est un résidu lysine et la séquence correspondant à celle du variant rHV2-Lys⁴⁷ de l'hirudine, ledit fragment d'ADN étant sous le contrôle du promoteur du gène MFα1 de la levure.

Enfin, la cassette d'expression selon l'invention peut comprendre, en outre, un terminateur de transcription fonctionnel dans la levure, par exemple, une séquence de terminateur du gène PGK.

La cassette d'expression selon l'invention peut être insérée dans un vecteur d'expression qui sert à transformer une cellule de levure hôte. Les vecteurs d'expression selon l'invention, seront en général des plasmides à replication autonome. Ces plasmides peuvent comporter des éléments assurant leur replication, c'est à dire une origine de replication, telle que celle du plasmide 2µ de levure et des éléments de sélection, tel le gène *ura*3 ou *leu*2 qui assure la complémentation des mutants de levure *ura*3 ou *leu*2*.* En particulier, on pourra avantageusement utiliser un gène de sélection délété de son promoteur afin d'augmenter le nombre de copies de plasmides dans une cellule de levure.

Ces plasmides peuvent également comporter des éléments assurant leur replication dans *Escherichia coli* (*E*. *coli*), lorsque le plasmide doit être un plasmide navette, par exemple, une origine de replication telle que celle du plasmide pBR322, un gène marqueur tel que le gène de la βlactamase conférant la résistance à l'ampicilline et/ou d'autres éléments connus de l'homme du métier.

De manière préférée, le vecteur d'expression selon l'invention comprend, en outre, une ou plusieurs copies de tout ou partie du gène KEX2 menant à une augmentation de l'activité protéolytique de l'endoprotéase yscF. Le gène complet pourvu de son propre promoteur peut être utilisé ainsi que des fragments de ce gène, comme cela est décrit dans la demande EPA 396436 au nom de la demanderesse.

L'invention s'étend également à une souche de levure transformée avec un vecteur d'expression selon l'invention ou comprenant une cassette d'expression selon l'invention intégrée dans son génome. Parmi toutes les souches de levure envisageables, on peut citer les levures *Saccharomyces cerevisiae*, *Hansenula polymorpha*, *Pichia pastoris* et *Kluyveromyces lactis*. Cependant, on utilisera de préférence les souches du genre *Saccharomyces,* notamment de l'espèce *cerevisiae.* On utilisera, par exemple, une souche de génotype *ura*3 ou *leu*2 ou autre qui pourra être complémentée par le vecteur selon l'invention, pour assurer le maintien du vecteur dans la levure par une pression de sélection appropriée. On pourra également utiliser une souche surproductrice de l'endoprotéase yscF comprenant dans son génome une ou plusieurs copies de tout ou partie du gène KEX2.

L'invention a également trait à un procédé de préparation par des levures d'une protéine hétérologue mature, selon lequel on cultive la souche de levure selon l'invention dans un milieu de culture approprié et on récupère la protéine hétérologue mature secrétée dans le milieu de culture.

Enfin l'invention a pour objet une protéine hétérologue mature obtenue par la mise en oeuvre d'un procédé selon l'invention.

Les exemples ci-après permettront de mettre en évidence d'autres caractéristiques et avantages de la présente invention. Ces exemples seront illustrés par les figures suivantes.

La Figure 1 représente la reconstitution du gène synthétique de la défensine A d'insecte par assemblage de 9 oligonucléotides.

La Figure 2 représente le profil de chromatographie obtenu après analyse par HPLC analytique en phase réverse, des surnageants de culture de **A**: la souche de *S*. *cerevisiae* TGY47.1 transformée par pTG6823 ; et **B**: la souche de *S*. *cerevisiae* TGY47.1 transformée par pTG6824.

La Figure 3 représente le profil de chromatographie obtenu après analyse par HPLC analytique en phase réverse, des surnageants de culture de **A**: la souche de *S*. *cerevisiae* TGY47.1 transformée par pTG8835 ; et **B**: la souche de *S*. *cerevisiae* TGY47.1 transformée par pTG8836.

La Figure 4 montre la séparation sur gel SDS-PAGE 12,5% des échantillons issus des cultures de **2** : la souche *S. cerevisiae* TGY47.1 non transformée ; **3** : la souche *S. cerevisiae* TGY47.1 transformée par le plasmide pTG8840 ; et **4** : la souche *S. cerevisiae* TGY47.1 transformée par le plasmide pTG8843. La ligne **1** correspond aux protéines marqueur, dont le poids moléculaire en kDA est indiqué sur le côté gauche de la Figure. Le gel est coloré au bleu de Coommassie.

### EXEMPLE 1: construction des vecteurs pTG6823 et pTG6824 et production de rHV2-Lys⁴⁷ dans le milieu de culture par une souche de levure transformée par lesdits vecteurs.

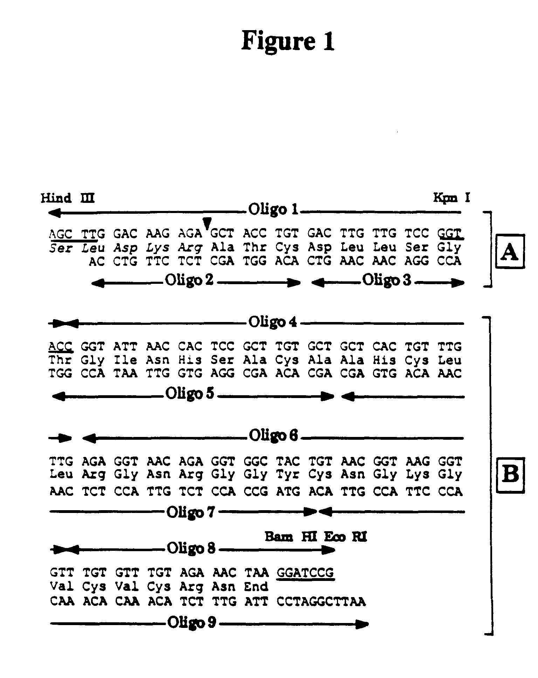

Le gène synthétique de la défensine A de *Phormia terranovae* est reconstitué en assemblant 9 oligonucléotides, comme indiqué dans la Figure 1.

La partie 5' du gène de la défensine A, à laquelle ont été rajoutés 15 nucléotides codant pour les 5 acides aminés C-terminaux de la séquence pro MFα1 de levure (Ser-Leu-Asp-Lys-Arg) dont le codon codant pour la sérine a été modifié pour générer en 5' un site *Hind*III*,* dérive de l'assemblage de trois oligonucléotides décrits dans les SEQ ID NO: 3, 4 et 5 (bloc A sur la Figure 1).

La partie 3' du gène de la défensine A d'insecte est obtenue par l'assemblage de six oligonucléotides décrits dans les SEQ ID NO: 6 à 11 (bloc B sur la Figure 1). Les oligonucléotides réassemblés sont introduits entre les sites *Kpn*I et *Eco*RI du phage M13TG131 (Kieny et al., Gene, 1983, 26, 91-99). La partie 5' est ensuite insérée entre les sites *Hind*III et *Kpn*I du vecteur phagique précédant, générant ainsi le phage M13TG3849 contenant le gène complet de la défensine A d'insecte

Le fragment *Hind*III*-Bam*HI comprenant la séquence codant pour la Ser-Leu-Asp-Lys-Arg-défensine A est isolé de M13TG3849. Parallèlement, un fragment *Sph*I-*Hind*III est purifié de M13TG3868. Le phage M13TG3868 contient la séquence codant pour une protéine de fusion composée de la séquence pré et la séquence pro MFα1 se terminant par le dipeptide Lys-Arg, suivie de la séquence correspondant à celle du variant rHV2-Lys⁴⁷ de l'hirudine, placée sous le contrôle du promoteur du gène de levure MFα1. On peut signaler que la séquence pro MFα1 contient un site artificiel *Hind*III au niveau du codon codant pour la sérine située 5 acides aminés avant l'extrémité C terminale de ladite séquence pro. De plus, le promoteur indu dans M13TG3868 est une version modifiée du promoteur du gène MFα1 (Inokuchi et al., Mol. Cell. Biol., 1987, *7*, 3185-3193). En effet, le promoteur est modifié par le remplacement du site *Eco*RI 5' par un site *Sph*I et par la destruction du site *Bgl*II interne par un traitement au grand fragment de l'ADN polymérase I d'*E*. *coli*. Le fragment *Sph*I-*Hind*III de M13TG3868 contient donc la séquence correspondant à la protéine de fusion suivante : séquence pré MFα1, séquence pro MFα1 dépourvue des 5 acides aminés C-terminaux Ser-Leu-Asp-Lys-Arg, le tout étant sous le contrôle du promoteur modifié du gène MFα1.

Les deux fragments sont purifiés sur gel et liés au vecteur M13mp18 (Gibco BRL) dans lequel le site unique *Hind*III est détruit par un traitement au grand fragment de l'ADN polymérase I d'*E*. *coli.* On transforme le mélange de ligation dans la souche *E*. *coli* NM522 et on identifie par digestion enzymatique un clone comprenant le promoteur modifié du gène MFα1 en amont de la séquence pré MFα1, de la séquence pro MFα1 se terminant par le dipeptide Lys-Arg suivie de la séquence synthétique codant pour la défensine A de *Phormia terranovae*. Ledit clone est désigné M13TG4813.

Le vecteur M13TG4813 est soumis à une mutagénèse dirigée mettant en oeuvre l'oligonucléotide décrit dans la SEQ ID NO: 12, afin d'introduire un site *Nhe*I dans la partie 3' de la séquence codant pour le peptide pré MFα1. Le phage ainsi modifié constitue le M13TG5879.

On isole un fragment *Sph*I*-Bam*HI de M13TG5879, ledit fragment contenant un bloc d'expression constitué du promoteur modifié du gène MFα1, et de la séquence codant pour une protéine de fusion comprenant la séquence pré MFα1, la séquence pro MFα1 et la séquence de la défensine A mature. On introduit le fragment purifié entre les sites *Sph*I et *Bam*HI du plasmide pTG3828 (Achstetter et al., Gene, 1992, *110*, 25-31). On transforme le mélange de ligation dans la souche *E*. *coli* 1106. On isole un clone présentant la cartographie de restriction attendue qui est appelé pTG5896. Ainsi, le bloc d'expression décrit ci-dessus est introduit dans un vecteur d'expression de la levure.

Le vecteur M13JM212 est un vecteur dérivé de M13mp18 comprenant la séquence ADNc naturelle codant pour la défensine A et une extension de 65 pb (Dimarcq et al., EMBO J., 1990, *9*, 2507-2515). A son extrémité 3', l'insert ADNc contient environ 60pb correspondant à la région non codante de l'ARNm de la défensine A. De plus, l'insert ADNc est muni à ses deux extrémités de linkers *Eco*RI. Le vecteur M13JM212 est soumis à une mutagénèse dirigée mettant en oeuvre l'oligonucléotide décrit dans la SEQ ID NO: 13. La séquence de l'ADNc est modifiée de manière à introduire un site de restriction *Hind*III donnant lieu au vecteur M13TG5824.

On reconstitue la séquence d'ADN codant pour la "version courte" de la séquence pro de la défensine A, débutant à l'acide aminé +6 et se terminant à l'acide aminé +31 de la SEQ ID NO: 2, ceci en mettant en oeuvre deux oligonucléotides synthétiques décrits dans les SEQ ID NO: 14 et 15. Une fois réassemblés, ces oligonucléotides forment un fragment *Nhe*I*-Afl*II. Le fragment synthétique inclut la jonction avec la séquence pré MFα1 ainsi que la jonction avec la séquence codant pour la défensine A mature.

Le fragment synthétique *Nhe*I-*Afl*II ainsi que le fragment *Afl*II*-Sal*I isolé de M13TG5824 sont liés avec le vecteur pTG5896 digéré par les enzymes *Nhe*I et *Sal*I. On transforme le mélange de ligation dans la souche *E*. *coli* 1106. On identifie un clone présentant un digest de restriction correct qui est désigné pTG5897. Le vecteur pTG5897 contient donc un bloc d'expression comprenant la protéine de fusion composée de la séquence pré MFα1, la séquence pro version courte du précurseur de la défensine A (acides aminés +6 à +31) et la défensine A mature, placée sous le contrôle du promoteur modifié du gène MFα1.

Ledit bloc d'expression est isolé de pTG5897 sous la forme d'un fragment *Sph*I*-Sal*I. On introduit ledit fragment dans le vecteur M13TG3868 digéré par les enzymes *Sph*I et *SalI,* générant ainsi le vecteur M13TG6801.

Le vecteur M13TG6801 est soumis à une mutagénèse dirigée afin de générer la "version longue" (acides aminés +1 à +31) de la séquence pro du précurseur de la défensine A, en mettant en oeuvre l'oligonucléotide décrit dans la SEQ ID NO: 16. Parallèlement au cours de la même réaction, on a également modifié la séquence correspondant à la jonction entre la séquence pro du précurseur de la défensine A et la séquence mature de la défensine A. On y a introduit 5 codons comprenant un site *Acc*I et codant pour les 5 acides aminés N-terminaux de rHV2-Lys⁴⁷, ceci en mettant en oeuvre l'oligonucléotide décrit dans la SEQ ID NO: 17. Le vecteur M13TG6806 ainsi généré, code pour la séquence pré MFα1, la séquence pro du précurseur de la défensine A (acides aminés +1 à +31), le peptide Ile-Thr-Tyr-Thr-Asp et la défensine A mature.

On isole du vecteur M13TG3868, un fragment *Acc*I*-Sal*I comportant la séquence codant pour rHV2-Lys⁴⁷ délétée des 5 paires de bases (pb) codant en partie pour le peptide Ile-Thr N-terminal. Le fragment est introduit dans le vecteur M13TG6806 digéré par *Acc*I et *Sal*I, donnant lieu à M13TG6807, qui contient le promoteur modifié du gène MFα1 précédant la protéine de fusion suivante : séquence pré MFα1, séquence pro du précurseur de la défensine A (acides aminés +1 à +31) et séquence du variant rHV2-Lys⁴⁷ de l'hirudine.

Ce bloc d'expression est isolé de M13TG6807 sous la forme d'un fragment *Sph*I*-Sal*I. Le fragment est inséré dans le vecteur d'expression de la levure pTG3828 digéré par les enzymes *Sph*I et *Sal*I, donnant lieu au vecteur pTG6823.

Parallèlement, le fragment *Sph*I*-Sal*I isolé de M13TG6807 est introduit entre les sites *Sph*I et *Sal*I du vecteur pTG4812. Le vecteur pTG4812 est dérivé du vecteur pTG3828, dans lequel on a introduit une copie du gène KEX2 de levure, ceci au niveau de la jonction de la partie 2µ et de la partie pBR322 du vecteur. On transforme le mélange de ligation dans la souche *E*. *coli* NM522 et on identifie un clone présentant un profil de digestion correct. Le vecteur pTG6824 ainsi généré, comprend la même cassette d'expression de rHV2-Lys⁴⁷ que pTG6823 ainsi qu'une copie du gène KEX2 insérée en dehors de ladite cassette d'expression.

On transforme les vecteurs d'expression pTG6823 et pTG6824 dans la souche *S*. *cerevisiae* TGY47.1 (MATα, pra1, prb1, prc1, cps1, ura3-delta5, leu2-3, leu2-112, his) ou dans la souche TGY48.1 qui dérive de la souche TGY47.1, mais présente une prototrophie pour la leucine (Leu+). Les cellules de levure sont transformées selon la technique standard de l'acétate de lithium (Ito et al., J. Bacteriol., 1983, *133*). D'autres protocoles de transformation de la levure peuvent également être employés. Des transformants prototrophes Ura+ sont sélectionnés sur milieu minimum (0,67% de bases azotées pour levure YNB (Yeast Nitrogen Base) sans acide aminé, 1% de casamino acides, 2% de glucose et 2% de bacto agar). On vérifie la stabilité du phénotype Ura+ sur boîte d'agar en milieu sélectif contenant le milieu décrit ci-dessus.

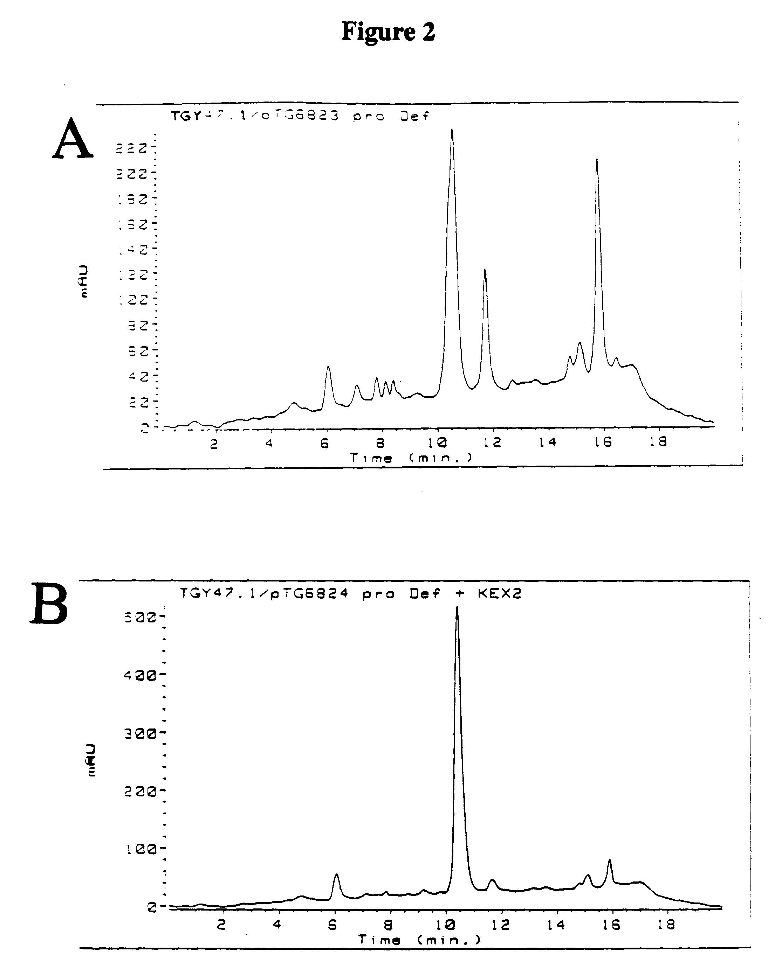

Des transformants TGY47.1/pTG6823 et TGY47.1/pTG6824 sont mis en culture en erlenmeyer à 28°C dans un milieu sélectif (0,67% de YNB sans acide aminé, 1% de casamino acides et 2% de glucose). Après 48h de culture, on sépare cellules et surnageants par filtration d'un aliquote de la culture sur membrane 0,22µ. Une fraction du surnageant est soumise à une HPLC (High Pressure Liquid Chromatography) analytique en phase réverse dans les conditions décrites dans Achstetter et al. (Gene, 1992, *110*, 25-31). On mesure l'absorbance de l'effluent à 205nm. Les profils de chromatographie obtenus pour les effluents issus de la culture TGY47.1/pTG6823 et TGY47.1/pTG6824 sont montrés dans les Figures 2A et 2B, respectivement.

On soumet le matériel élué de la colonne et présentant un temps de rétention d'environ 10,25min à un séquençage des acides aminés N-terminaux. Le matériel dérivé de la culture TGY47.1/pTG6823 montre un profil de chromatographie hétérogène dans lequel deux types de séquences N-terminales sont lues en quantité à peu près équivalente :
(1) la séquence décrite dans la SEQ ID NO: 18 où Xaa représente les acides aminés qui n'ont pas pu être identifiés clairement. Cette séquence correspond à la séquence N-terminale attendue pour le variant mature rHV2-Lys⁴⁷ et,
(2) la séquence décrite dans la SEQ ID NO: 19 où Xaa représente les acides aminés qui n'ont pas pu être identifiés clairement. Ladite séquence correspond à la séquence N-terminale du rHV2-Lys⁴⁷ comprenant en outre une extension N-terminale de trois acides aminés provenant de la séquence pro du précurseur de la défensine A d'insecte.

Par contre, avec le matériel provenant de la culture TGY47.1/pTG6824, on obtient un pic homogène et unique qui donne lieu à la séquence en acides aminés décrite dans la SEQ ID NO: 20, correspondant à la séquence N-terminale du rHV2-Lys⁴⁷ mature (Xaa représente les acides aminés qui n'ont pas pu être identifiés clairement). Ainsi, la coexpression du gène KEX2 produit une protéine hétérologue mature et homogène.

De plus, on détermine l'activité inhibitrice du variant rHV2-Lys⁴⁷ secrété vis à vis de la thrombine, en utilisant un test colorimétrique (activité protéolytique sur un substrat synthétique, le chromozyme TH, Boehringer Mannheim). Le tableau 1 présente les résultats des dosages. L'activité du rHV2-Lys⁴⁷ est exprimé en ATU/A₆₀₀ (1-2 x 10⁷ cellules/ml correspondent à 1 unité A₆₀₀nm).

**Tableau 1**

| Plasmides | KEX2 | rHV2-Lys⁴⁷ (ATU/A₆₀₀nm) |
|---|---|---|
| pTG6823 | - | 8,6 |
| pTG6824 | + | 25,6 |

On peut voir que la présence du gène KEX2 dans le plasmide pTG6824 permet une augmentation d'un facteur 3 de la production de rHV2-Lys⁴⁷ sous forme active.

### EXEMPLE 2 : construction des vecteurs pTG8835 et pTG8836 et production de rHV2-Lys⁴⁷ dans le milieu de culture d'une souche de levure transformée par lesdits vecteurs.

Le vecteur M13TG6807 est soumis à une mutagénèse dirigée afin de remplacer le résidu alanine en position +9 de la séquence pro "version longue" de la défensine A par un résidu thréonine, en mettant en oeuvre l'oligonucléotide décrit dans la SEQ ID NO: 21. Le phage muté est vérifié par séquençage et donne lieu au M13TG8832.

La cassette d'expression comportant le promoteur modifié du gène MFα1 dirigeant l'expression de la séquence codant pour la séquence pré MFα1, la séquence pro "version longue" (Ala⁹ -> Thr) du précurseur de la défensine A de *Phormia terranovae* et rHV2-Lys⁴⁷ mature, est isolée de M13TG8832 sous la forme d'un fragment *Sph*I*-Sal*I. Le fragment purifié est inséré dans le vecteur pTG3828 digéré par les enzymes *Sph*I et *Sal*I, donnant lieu au vecteur pTG8835.

Parallèlement, le fragment *Sph*I*-Sal*I purifié de M13TG8832 est également cloné entre les sites *Sph*I et *Sal*I du vecteur pTG4812, lequel contient une copie du gène KEX2 de levure.

On transforme les plasmides pTG8835 et pTG8836 dans les cellules de levure et on analyse les transformants obtenus selon le protocole décrit précédemment.

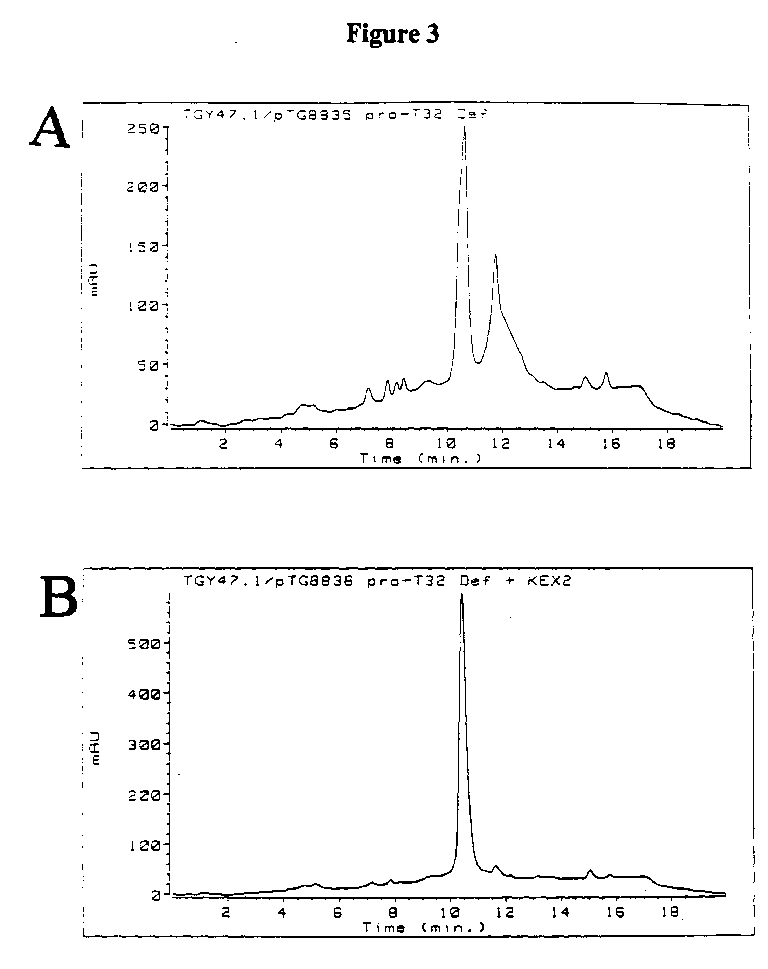

Les profils chromatographiques obtenus sont présentés dans les Figures 3A et 3B, correspondant respectivement aux effluents issus de la culture TGY47.1/pTG8835 et TGY47.1/pTG8836. Les résultats des dosages de l'activité antithombine sont présentés dans le tableau 2

**Tableau 2**

| Plasmides | KEX2 | rHV2-Lys⁴⁷ (ATU/A₆₀₀nm) |
|---|---|---|
| pTG8835 | - | 10,4 |
| pTG8836 | + | 25,6 |

On peut constater que la présence du gène KEX2 dans la construction selon l'invention (pTG8836) réduit le nombre de pics générés lors de l'analyse des surnageants de culture par chromatographie et améliore la production de rHV2-Lys⁴⁷ active d'un facteur 2,5 fois. Par contre, la mutation de l'alanine en position +9 de la séquence pro du précurseur de la défensine A d'insecte en thréonine, ne produit qu'un effet mineur sur la qualité et la quantité de rHV2-Lys⁴⁷ produit.

### EXEMPLE 3 : construction des vecteurs pTG8840 et pTG8843 et production de l'Ala-Pro-trophoblastine ovine dans le milieu de culture d'une souche de levure transformée par lesdits vecteurs.

Le vecteur M13TG7720 (Degryse et al, Gene, 1992, *118,* 47-53) est digéré par les enzymes *Hind*III et *Eco*RI puis traité par le grand fragment de l'ADN polymérase I d'E. *coli*. Le fragment de 0,5kb résultant de ce traitement, est purifié sur gel d'agarose et inséré dans le vecteur M13TG6807, préalablement digéré par *Acc*I et traité par le grand fragment de l'ADN polymérase I *d'*E. *coli*. On transforme le mélange de ligation dans la souche *E*. *coli* NM522 et on identifie un phage ayant incorporé le fragment de 0,5kb. Le clone est désigné M13TG8818.

Le vecteur M13TG8818 est soumis à une mutagénèse dirigée afin d'introduire en amont de la séquence codant pour l'extrémité N-terminale de la trophoblastine ovine, une séquence codant pour le dipeptide Ala-Pro, en mettant en oeuvre l'oligonucléotide décrit dans la SEQ ID NO: 22. Après mutagénèse, la séquence des clones générés est vérifiée. On choisit un clone contenant la mutation désirée, désigné M13TG8834.

Le vecteur M13TG8834 est à son tour modifié par mutagénèse dirigée, mettant en oeuvre l'oligonucléotide décrit dans la SEQ ID NO: 23. La mutagénèse permet de générer une jonction correcte entre les séquences codantes pour l'extrémité 3' de la séquence pro du précurseur de la défensine A de *Phormia terranovae* et la protéine hétérologue Ala-Pro-trophoblastine. On identifie un clone muté par séquençage, donnant lieu au vecteur M13TG8838. Ce dernier contient une cassette d'expression composée de la version modifiée du promoteur du gène MFα1 en amont de la séquence codant pour une protéine de fusion composée de la séquence pré MFα 1, la séquence pro du précurseur de la défensine A de *Phormia terranovae* (31 acides aminés) et l'Ala-Pro-trophoblastine.

On digère le vecteur M13TG8838 par l'enzyme *Eco*RI et on le traite par le grand fragment de l'ADN polymérase I d'*E*. *coli*. Le fragment ainsi généré, contenant la cassette d'expression décrite ci-dessus, est purifié et inséré dans le vecteur pTG3828 linéarisé par *Sma*I. On identifie un transformant ayant intégré la cassette d'expression dans l'orientation correcte par rapport au terminateur PGK. La construction est désignée pTG8840.

Parallèlement, le fragment *Eco*RI traité de manière à avoir les extrémités franches et isolé de M13TG8838, est également inséré dans le vecteur pTG4812 linéarisé par *Sma*I. On identifie par analyse de restriction, un clone présentant la cassette d'expression dans l'orientation correcte vis à vis du terminateur PGK. Le clone est appelé pTG8843.

On transforme les vecteurs d'expression pTG8840 et pTG8843 dans la souche de levure TGY47.1, selon la méthode décrite précédemment.

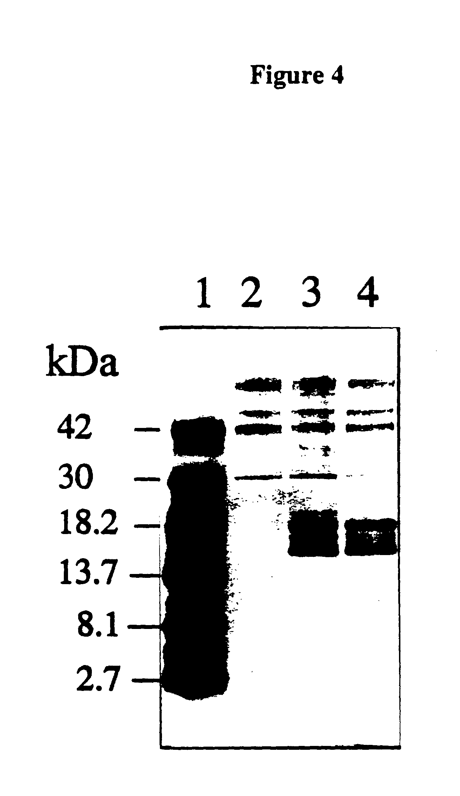

Des transformants de chaque type sont mis en culture en erlenmeyer à 28°C dans un milieu sélectif (0,67% de YNB sans acide aminé, 1% de casamino acides et 2% de glucose). Après 48h de culture, on sépare les cellules et surnageants par filtration d'une fraction de la culture sur membrane 0,22µ. Le surnageant obtenu est concentré par ultrafiltration (Centricon YM5, seuil de coupure 5kDa). On soumet un aliquote du filtrat obtenu à une électrophorèse en gel de polyacrylamide-SDS (SDS-PAGE). L'échantillon est chauffé à 95°C pendant 5min avant d'être chargé sur un gel de polyacrylamide 12,5%. Après migration, le gel est coloré par le bleu de Coomassie pour visualiser l'ensemble des protéines (Figure 4). Dans le matériel provenant des levures transformées par les vecteurs pTG8840 et pTG8843, on visualise un doublet de bandes, l'une à 15kDa et l'autre présentant une mobilité électrophorétique légèrement supérieure. De plus, on détecte également d'autres bandes présentant une mobilité électrophorétique inférieure, qui ne sont pas non plus présentes dans l'échantillon correspondant au contrôle négatif. L'ensemble des bandes spécifiquement détectées dans les échantillons pTG8840 et pTG8843, est en outre révélé en Western blot par un antisérum de lapin anti-trophoblastine ovine. Le matériel de mobilité électrophorétique plus élevée peut être dû à des produits de dégradation de la trophoblastine ou à un artéfact d'electrophorèse. Par contre, le matériel de mobilité électrophorétique inférieure, reconnu par les anticorps de lapin anti-trophoblastine ovine, peut être dû à des aggrégats protéiques qui peuvent se former malgré le traitement des échantillons à 95°C.

Les bandes formant un doublet à environ 15 à 18 kDa sont transférées sur une membrane PVDF (polyvinyliden difluoride). On soumet le matériel transféré à une analyse de la séquence en acides aminés N-terminaux. On obtient une seule séquence en acides aminés décrite dans la SEQ ID NO: 24, qui correspond à la séquence attendue pour une protéine hétérologue Ala-Pro-trophoblastine et où Xaa représente les acides aminés qui n'ont pu être identifiés clairement.

### EXEMPLE 4 : vecteurs d'expression de rHV2-Lys⁴⁷ dans lesquels la séquence pro de la défensine A comprend un résidu cystéine en position 2, 6, 9 ou 17.

Le vecteur M13TG6807 est soumis à une mutagénèse dirigée afin de remplacer le résidu proline en position +2 ou l'un des résidus alanine en position +6, +9 ou +17 par un résidu cystéine. On met en oeuvre les oligonucléotides mutagènes décrits respectivement dans les SEQ ID NO: 25 à 28. On obtient les vecteurs M13TG9836, M13TG9838, M13TG9839 et M13TG9840.

Un fragment *Sph*I*-Sal*I de 1,4 kb est isolé de chacun des vecteurs précédents avant d'être inséré soit dans le vecteur pTG3828 ou pTG4812 également digéré par *Sph*I et *Sal*I. On transforme les vecteurs obtenus dans les cellules de levure et on évalue l'activité antithrombine dans les surnageants de culture comme indiqué précédemment.

L'activité antitrombine de la rHV2Lys⁴⁷ secrétée des constructions mettant en oeuvre les variants Cys⁺², Cys⁺⁶, Cys⁺⁹ et Cys⁺¹⁷ de la séquence pro défensine A est comparable à celle obtenue avec la séquence pro de type sauvage.

Comme précédemment, on observe une production de rHV2-Lys⁴⁷ active améliorée d'un facteur 2,5 à 3 fois dans les constructions dérivées de pTG4812 qui incluent le gène KEX2.

### EXEMPLE 5 : construction de vecteurs comprenant la séquence pré BGL2, la séquence pro de la défensine A dans laquelle le résidu arginine en position 28 est remplacé par un résidu proline (Arg²⁸ -> Pro) et la séquence codante pour rHV2-Lys⁴⁷, sous le contrôle du promoteur modifié du gène MFα1.

On isole un fragment *Pst*I de M13TG4815. Celui-ci comprend le promoteur MFα1 modifié mais incluant en outre un site *Xho*I en position -12 par rapport à l'ATG initiateur (*Xho*I-12) suivi de la séquence pré BGL2, de la séquence pro MFα1 et de la séquence synthétique codant pour la défensine A de *Phormia terranovae*. La séquence pré BGL2 est décrite dans Klebl et Tanner, (J. Bacteriol., 1989, *171,* 6259-6264). Ce fragment *PstI* de 768pb qui porte le promoteur MFα1 tel que décrit ci-dessus ainsi que les 8 premiers acides aminés de la séquence pré BGL2, est sous cloné dans le vecteur M13TG4813 digéré par *Pst*I. Il en résulte M13TG5871.

On isole le fragment *Sph*I*-Bam*HI de M13TG5871 comprenant le promoteur MFα1 (*Xho*I-12), une séquence pré de fusion composée des résidus 1 à 8 de la séquence pré BGL2 et des résidus 8 à 18 de la séquence MFα1, la séquence pro MFα 1 et le gène synthétique défensine A. Ce fragment est introduit dans pTG3828 également traité par les enzymes *Sph*I et *Bam*HI pour donner pTG5885.

Par ailleurs, le vecteur M13TG6801, déjà mentionné, est soumis à une mutagénèse dirigée afin de générer la version longue (acides aminés +1 à +31) de la séquence pro de la défensine A à l'aide de l'oligonucléotide OTG3051 (SEQ ID NO: 29). Par la même occasion, on introduit un site *Nhe*I en 3' de la séquence pré MFα1. On obtient pTG6820. On introduit le fragment *Sph*I*-Bam*HI purifié de pTG6820 entre les mêmes sites de pTG3828, pour générer pTG6821.

pTG5885 est digéré par les enzymes *Xho*I et *Sal*I puis ligé en présence de deux fragments, l'un *Xho*I*-Nhe*I provenant de la rehybridation des oligonucléotides synthétiques OTG3070 et OTG3071 (SEQ ID NO: 30 et 31) et l'autre *Nhe*I*-Sal*I isolé de pTG6821 et correspondant à l'extremité 3' de la séquence pré de la défensine A, la séquence pro (version longue) et la séquence de la défensine A mature. On obtient pTG6880.

D'autre part, le vecteur pTG3828 est digéré partiellement par *Xba*I, traité par le grand fragment Klenow de l'ADN polymérase I d'*E*. *coli* avant d'être religué, donnant lieu à pTG6888 dans lequel le site *Xba*I situé dans le 2µ est détruit. Celui-ci est digéré par *Sph*I et *Bam*HI, sites entre lesquels on introduit le fragment *Sph*I*-Bam*HI isolé de M13TG8883. Ce dernier comprend une cassettte d'expression composée du promoteur modifié du gène MFα1, dirigeant l'expression d'un précurseur "séquence pré BGL-2, séquence pro MFα1, séquence codant pour la protéine mature OspA" (Bergstrom et al., Mol Microbiol., 1989, *3*, 479-486). On obtient pTG8890.

On amplifie par PCR à partir de la matrice pTG8890 et des amorces OTG4442 et OTG4443 (SEQ ID N0: 32 et 33) un fragment portant le promoteur modifié du gène MFα1 suivi de la séquence pré BGL2.

Parallèlement on amplifie, également par PCR, un fragment comportant la séquence pro de la défensine A à partir de pTG6880 et des amorces OTG4444 (SEQ ID NO: 34) et OTG4445 (SEQ ID NO: 35). Les amorces permettent d'introduire également des mutations silencieuses pour générer un site *Eag*I près de l'extrémité 3' de la séquence pro et un site *Kpn*I au delà de cette extremité. Puis on assemble les deux fragments PCR pour former un fragment linéaire *Sph*I*-Kpn*I par un double PCR à l'aide des oligonucléotides OTG4442 (SEQ ID NO: 32) et OTG 4445 (SEQ ID NO: 35). Il est inséré dans le vecteur M13TG131 également traité par *Sph*I et *Kpn*I pour donner M13TG8898.

On soumet celui-ci à une mutagénèse dirigée pour corriger une mutation A -> G en position -5 par rapport à l'ATG initiateur. On met en oeuvre l'oligonucléotide OTG4637 (SEQ ID NO: 36) pour générer M13TG9800.

On isole par PCR la séquence codante pour rHV2-Lys⁴⁷, munie d'une extension N-terminale correspondant aux derniers acides aminés de la séquence pro de la défensine A, dont les codons codant pour les résidus en position 27 et 28 ont été modifiés silencieusement de manière à introduire un site de restriction *Eag*I. D'autre part, on introduit en 3' du codon stop de rHV2-Lys⁴⁷ un site *Sal*I. On utilise à titre de matrice le vecteur M13TG6807 et les deux amorces OTG5074 et OTG5075 reportées dans les SEQ ID NO: 37 et 38.

On introduit le fragment *Eag*I*-Sal*I généré par PCR entre les mêmes sites de M13TG 9800. On obtient M13TG9853 lequel est digéré par *Sal*I puis traité par le grand fragment de l'ADN polymérase I d'*E*. *coli*, avant d'être religé sur lui-même (destruction du site *Sal*I). On génère M13TG9854.

Ce dernier est clivé par *EagI* et *Acc*I puis ligué à un adaptateur constitué des 2 oligonucléotides OTG5136 et OTG5137 rehybridés (SEQ ID NO: 39 et 40). Après transformation du mélange de ligation dans *E*. *coli*, on isole quelques clones dont on vérifie la séquence par les techniques standards. On sélectionne un clone dont la séquence est correcte, désigné ci-après M13TG9856.

On isole le fragment *Sph*I*-Bgl*II de 1,4 kb de M13TG9856 lequel est inséré entre les sites *Sph*I et *Bgl*II de pTG3828 ou *Sph*I et *Bam*HI de pTG4812, pour donner respectivement pTG9860 et pTG9863.

Comme précédemment, après transformation de ces 2 vecteurs d'expression dans la souche de levure TGY47.1, on détermine l'activité antithrombine de l'hirudine rHV2-Lys⁴⁷ secrétée dans le surnageant des cultures correspondantes. L'activité mesurée à partir des constructions comprenant la séquence pro (Arg²⁸ -> Pro) de la défensine A est comparable à celle obtenue avec les vecteurs incluant une séquence pro de type sauvage. On observe également une amélioration d'un facteur 3 lorsque le vecteur inclut le gène KEX2 (pTG9863 versus pTG9860).

### EXEMPLE 6 : construction du vecteur pTG8709 et production de la xénoxine-1 dans le milieu de culture d'une souche de levure transformée par ledit vecteur.

On amplifie par PCR la séquence codant pour la xénoxine-1 mature munie d'un site *Eag*I en 5' et d'un site *Sal*I en 3'. On utilise à titre de matrice M13TG4414 ou pTG4414 (Kolbe et al., J. Biol. Chem., 1993, *268*, 16458-16464) et les amorces OTG5770 et OTG5771 (SEQ ID NO: 41 et 42). Le fragment ainsi généré est intégré dans M13TG9800 préalablement digéré par *Eag*I et *Sali* pour donner M13TG8703. Celui-ci comprend une cassette d'expression composée du promoteur MFα1 modifié dirigeant l'expression d'un précurseur "séquence pré BGL2, séquence pro MFα1, séquence codant pour la xénoxine-1 mature". La séquence en acide aminé de cette dernière est indiquée dans la SEQ ID NO: 43.

On introduit le fragment *Sph*I*-Sal*I isolé de M13TG8703 et portant la cassette d'expression entre les même sites de pTG4812. On obtient pTG8709 qui est transformé dans la souche *S*. *cerevisiae* TGY73.4 laquelle dérive de TGY47.1 mais présente une prototrophie pour la leucine (Leu⁺). On sélectionne un transformant Ura⁺ que l'on fait pousser en milieu sélectif (décrit dans l'exemple 1) pendant 60 heures. Le surnageant de culture est concentré par ultrafiltration sur membrane YM2 et centrifugation selon les techniques conventionnelles. Le concentré est soumis à une electrophorèse dénaturante sur gel de polyacrylamide (15,3 % en polyacrylamide). Les protéines sont transférées sur membrane PVDF et le matériel correspondant au poids moléculaire attendu (environ 7kDa) est soumis à un séquençage des acides aminés N-terminaux. On lit une séquence, présente en quantité majoritaire, qui correspond à la séquence N-terminale attendue pour la xénoxine-l mature ; (SEQ ID NO: 44 ; Xaa représente les acides aminés qui n'ont pas pu être identifiés clairement).

## Revendications

1. Une cassette d'expression permettant la synthèse d'une protéine hétérologue mature, à l'exclusion de la défensine de Phormia terranovae, et comprenant au moins un fragment d'ADN codant pour un précurseur de ladite protéine hétérologue ; ledit précurseur comprenant :
- une séquence pré fonctionnelle dans la levure,
- la séquence pro du précurseur de la défensine A de *Phormia terranovae*, un variant ou un fragment fonctionnel de celle-ci, et
- une séquence correspondant à celle de ladite protéine hétérologue sous forme mature ;
ledit fragment d'ADN étant sous le contrôle des éléments nécessaires à son expression.

2. Une cassette d'expression selon la revendication 1, caractérisée en ce que le fragment d'ADN code pour un précurseur comprenant une séquence pré qui est celle du précurseur du facteur a de la levure *Saccharomyces cerevisiae*.

3. Une cassette d'expression selon la revendication 1 ou 2, caractérisée en ce que la séquence pro a une séquence en acides aminés telle que montrée dans la SEQ ID NO:1, débutant à l'acide aminé en position +1 ou +6 et se terminant à l'acide aminé +31, dans laquelle, ensemble ou séparément, le résidu Xaa en position +2 est un résidu proline ou cystéine, le résidu Xaa en position +6 est un résidu alanine ou cystéine, le résidu Xaa en position +9 est un résidu alanine cystéine ou thréonine, le résidu Xaa en position +17 est un résidu alanine ou cystéine, le résidu Xaa en position +28 est un résidu arginine ou proline et le résidu Xaa en position +30 est un résidu arginine ou lysine.

4. Une cassette d'expression selon l'une des revendications 1 à 3, caractérisée en ce que la séquence pro a la séquence en acides aminés telle que montrée dans la SEQ ID N0: 2.

5. Une cassette d'expression selon l'une des revendications 1 à 4, caractérisée en ce que le fragment d'ADN comporte une séquence correspondant à celle de l'hirudine.

6. Une cassette d'expression selon la revendication 5, caractérisée en ce que le fragment d'ADN comporte une séquence correspondant à celle du variant rHV2-Lys⁴⁷ de l'hirudine.

7. Une cassette d'expression selon l'une des revendications 1 à 4, caractérisée en ce que le fragment d'ADN comporte une séquence correspondant à celle de la trophoblastine ovine comprenant à son extrémité 5' une extension constituée par le dipeptide Ala-Pro.

8. Une cassette d'expression selon l'une des revendications 1 à 4, caractérisée en ce que le fragment d'ADN comporte une séquence correspondant à celle d'un peptide de la famille des xénoxines.

9. Une cassette d'expression selon l'une des revendications 1 à 8, caractérisée en ce que ladite cassette d'expression comprend en outre un terminateur de transcription fonctionnel dans la levure.

10. Un vecteur d'expression comprenant une cassette d'expression selon l'une quelconque des revendications 1 à 9.

11. Un vecteur d'expression selon la revendication 10, caractérisé en ce qu'il comprend en outre une ou plusieurs copies de tout ou partie du gène KEX2.

12. Une souche de levure comprenant une cassette d'expression selon l'une quelconque des revendications 1 à 9 ou un vecteur d'expression selon la revendication 10 ou 11.

13. Une souche de levure selon la revendication 12, caractérisée en ce que ladite souche est sélectionnée parmi les levures *Saccharomyces cerevisiae*, *Hansenula polymorpha*, *Pichia pastoris et Kluyveromyces lactis.*

14. Procédé de préparation d'une protéine hétérologue, selon lequel :
- on cultive une levure selon la revendication 12 ou 13 dans un milieu approprié et,
- on récupère la protéine hétérologue dans le milieu de culture sous forme mature.

## Patentansprüche

1. Expressionskassette, welche die Synthese eines reifen heterologen Proteins, mit Ausnahme des Defensins von *Phormia terranovae*, ermöglicht, und welche wenigstens ein DNA-Fragment enthält, welches für einen Vorläufer dieses heterologen Proteins kodiert, wobei der Vorläufer
- eine in der Hefe funktionelle Prä-Sequenz,
- die Pro-Sequenz des Vorläufers des Defensins A von *Phormia terranovae,* einer Variante oder eines funktionellen Fragments davon, und
- eine Sequenz, welche der des heterologen Proteins in reifer Form entspricht,
enthält, wobei das DNA-Fragment unter der Kontrolle von Elementen steht, die für seine Expression notwendig sind.

2. Expressionskassette gemäß Anspruch 1, dadurch gekennzeichnet, dass das DNA-Fragment für einen Vorläufer kodiert, welcher eine Prä-Sequenz enthält, die der des Vorläufers des a-Faktors der Hefe *Saccharomyces cerevisiae* entspricht.

3. Expressionskassette gemäß Anspruch 1 oder 2, dadurch gekennzeichnet, dass die Pro-Sequenz die unter SEQ ID NO:1 aufgeführte Aminosäuresequenz, beginnend mit der Aminosäure an Position +1 oder +6 und endend mit der Aminosäure +31, aufweist, in der, zugleich oder einzeln, der Rest Xaa an Position +2 ein Prolin- oder Cysteinrest ist, der Rest Xaa an Position +6 ein Alanin- oder Cysteinrest ist, der Rest Xaa an Position +9 ein Alanin-, Cystein- oder Threoninrest ist, der Rest Xaa an Position +17 ein Alanin- oder Cysteinrest ist, der Rest Xaa an Position +28 ein Arginin- oder Prolinrest ist und der Rest Xaa an Position +30 ein Arginin- oder Lysinrest ist.

4. Expressionskassette gemäß einem der Ansprüche 1 bis 3, dadurch gekennzeichnet, dass die Pro-Sequenz die unter SEQ ID NO:2 aufgeführte Aminosäuresequenz aufweist.

5. Expressionskassette gemäß einem der Ansprüche 1 bis 4, dadurch gekennzeichnet, dass das DNA-Fragment eine Sequenz aufweist, die der des Hirudins entspricht.

6. Expressionskassette gemäß Anspruch 5, dadurch gekennzeichnet, dass das DNA-Fragment eine Sequenz aufweist, die der der rHV2-Lys⁴⁷-Variante des Hirudins entspricht.

7. Expressionskassette gemäß einem der Ansprüche 1 bis 4, dadurch gekennzeichnet, dass das DNA-Fragment eine Sequenz aufweist, die der des Schafstrophoblastins entspricht, welche an ihrem 5'-Ende eine durch das Dipeptid Ala-Pro gebildete Verlängerung enthält.

8. Expressionskassette gemäß einem der Ansprüche 1 bis 4, dadurch gekennzeichnet, dass das DNA-Fragment eine Sequenz aufweist, die der eines Peptids der Xenoxin-Familie entspricht.

9. Expressionskassette gemäß einem der Ansprüche 1 bis 8, dadurch gekennzeichnet, dass die Expressionskassette außerdem einen in der Hefe funktionellen Transkriptionsterminator enthält.

10. Expressionsvektor, welcher eine Expressionskassette gemäß wenigstens einem der Ansprüche 1 bis 9 enthält.

11. Expressionsvektor gemäß Anspruch 10, dadurch gekennzeichnet, dass er außerdem eine oder mehrere vollständige oder teilweise Kopien des Gens KEX2 enthält.

12. Hefestamm, welcher eine Expressionskassette gemäß wenigstens einem der Ansprüche 1 bis 9 oder einen Expressionsvektor gemäß Anspruch 10 oder 11 enthält.

13. Hefestamm gemäß Anspruch 12, dadurch gekennzeichnet, dass es sich um einen der Hefestämme *Saccharomyces cerevisiae, Hansenula polymorpha*, *Pichia pastoris* und/oder *Kluyveromyces lactis* handelt.

14. Verfahren zur Herstellung eines heterologen Proteins, in dem man
- eine Hefe gemäß Anspruch 12 oder 13 in einem geeigneten Medium kultiviert und
- das heterologe Protein aus dem Kulturmedium in reifer Form gewinnt.

## Claims

1. Expression cassette allowing the synthesis of a mature heterologous protein, with the exception of *Phormia terranovae* defensin, and comprising at least one DNA fragment encoding a precursor of the said heterologous protein; the said precursor comprising:
- a pre sequence which is functional in yeast,
- the pro sequence of the precursor of *Phormia terranovae* defensin A, a variant or a functional fragment of the latter, and
- a sequence corresponding to that of the said heterologous protein in a mature form;
the said DNA fragment being under the control of elements necessary for its expression.

2. Expression cassette according to Claim 1, characterized in that the DNA fragment encodes a precursor comprising a pre sequence which is that of the precursor of factor α from the yeast *Saccharomyces cerevisiae.*

3. Expression cassette according to Claim 1 or 2, characterized in that the pro sequence has an amino acid sequence as shown in SEQ ID NO: 1, starting with the amino acid in position +1 or +6 and ending with the amino acid +31, in which, together or separately, the Xaa residue in position +2 is a proline or cysteine residue, the Xaa residue in position +6 is an alanine or cysteine residue, the Xaa residue in position +9 is an alanine, cysteine or threonine residue, the Xaa residue in position +17 is an alanine or cysteine residue, the Xaa residue in position +28 is an arginine or proline residue and the Xaa residue in position +30 is an arginine or lysine residue.

4. Expression cassette according to one of Claims 1 to 3, characterized in that the pro sequence has the amino acid sequence as shown in SEQ ID NO: 2.

5. Expression cassette according to one of Claims 1 to 4, characterized in that the DNA fragment comprises a sequence corresponding to that of hirudin.

6. Expression cassette according to Claim 5, characterized in that the DNA fragment comprises a sequence corresponding to that of the variant rHV2-Lys⁴⁷ of hirudin.

7. Expression cassette according to one of Claims 1 to 4, characterized in that the DNA fragment comprises a sequence corresponding to that of ovine trophoblastin comprising at its 5' end an extension consisting of the dipeptide Ala-Pro.

8. Expression cassette according to one of Claims 1 to 4, characterized in that the DNA fragment comprises a sequence corresponding to that of a peptide of the xenoxin family.

9. Expression cassette according to one of Claims 1 to 8, characterized in that the said expression cassette comprises, in addition, a transcription terminator which is functional in yeast.

10. Expression vector comprising an expression cassette according to any one of Claims 1 to 9.

11. Expression vector according to Claim 10, characterized in that it comprises, in addition, one or more copies of all or part of the KEX2 gene.

12. Yeast strain comprising an expression cassette according to any one of Claims 1 to 9 or an expression vector according to Claim 10 or 11.

13. Yeast strain according to Claim 12, characterized in that the said strain is selected from the yeasts *Saccharomyces cerevisiae, Hansenula polymorpha*,, *Pichia pastoris* and *Kluyveromyces lactis.*

14. Process for the preparation of a heterologous protein, according to which:
- a yeast according to Claim 12 or 13 is cultured in an appropriate medium and,
- the heterologous protein is recovered from the culture medium in a mature form.


### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67000
      (G) TELEPHONE: (33) 88 27 91 00
      (H) TELECOPIE: (33) 88 22 58 07
   (ii) TITRE DE L' INVENTION: Nouvelle sequence pro permettant la secretion de proteines heterologues a partir d'une cellule de levure.
   (iii) NOMBRE DE SEQUENCES: 44
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
   (vi) DONNEES DE LA DEMANDE ANTERIEURE:
      (A) NUMERO DE DEPOT: FR 90 00171
      (B) DATE DE DEPOT: 11-JAN-1993
(2) INFORMATION POUR LA SEQ ID NO: 1:
   (i) CARACTERISTIQUES DE LA SEQUENCE:
      (A) LONGUEUR: 31 acides aminés
      (B) TYPE: acide aminé
      (D) CONFIGURATION: linéaire
   (ii) TYPE DE MOLECULE: peptide
   (iii) HYPOTHETIQUE: NON
   (iii) ANTI-SENS: NON
   (v) TYPE DU FRAGMENT: interne
   (vi) ORIGINE:
      (A) ORGANISME: sequence pro de la defensine A
      (B) SOUCHE: Phormia terranovae
   (xi) DESCRIPTION DE LA SEQUENCE: SEQ ID NO: 1:
   Ile Xaa Ala Asp Ala Xaa Asn Asp Xaa His Phe Val Asp Gly Val Gln
   1               5                   10                  15
   Xaa Leu Lys Glu Ile Glu Pro Glu Leu His Gly Xaa Tyr Xaa Arg
               20                  25                  30
(2) INFORMATION POUR LA SEQ ID NO: 2:
   (i) CARACTERISTIQUES DE LA SEQUENCE:
      (A) LONGUEUR: 31 acides aminés
      (B) TYPE: acide aminé
      (D) CONFIGURATION: linéaire
   (ii) TYPE DE MOLECULE: peptide
   (iii) HYPOTHETIQUE: NON
   (iii) ANTI-SENS: NON
   (v) TYPE DU FRAGMENT: interne
   (vi) ORIGINE:
      (A) ORGANISME: sequence pro de la defensine A de Phormia terranovae
      (B) SOUCHE: Phormia terranovae
   (xi) DESCRIPTION DE LA SEQUENCE: SEQ ID NO: 2:
   Ile Pro Ala Asp Ala Ala Asn Asp Ala His Phe Val Asp Gly Val Gln
   1               5                   10                  15
   Ala Leu Lys Glu Ile Glu Pro Glu Leu His Gly Arg Tyr Lys Arg
               20                  25                  30
(2) INFORMATION POUR LA SEQ ID NO: 3:
   (i) CARACTERISTIQUES DE LA SEQUENCE:
      (A) LONGUEUR: 41 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 3:
   AGCTTGGACA AGAGAGCTAC CTGTGACTTG TTGTCCGGTA C                41
(2) INFORMATION POUR LA SEQ ID NO: 4:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovaea
      (C) INDIVIDUEL ISOLE: oligonucleotide de reconstitution du gene defensine A
   (xi) DESCRIPTION DE LA SEQUENCE: SEQ ID NO: 4:
   ACAGGTAGCT CTCTTGTCCA                                        20
(2) INFORMATION POUR LA SEQ ID NO: 5:
   (i) CARACTERISTIQUES DE LA SEQUENCE:
      (A) LONGUEUR: 13 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 5:
   CGGACAACAA GTC                                                    13
(2) INFORMATION POUR LA SEQ ID NO: 6:
   (i) CARACTERISTIQUES DE LA SEQUENCE:
      (A) LONGUEUR: 40 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 6:
   CGGTATTAAC CACTCCGCTT GTGCTGCTCA CTGTTTGTTG                       40
(2) INFORMATION POUR LA SEQ ID NO: 7:
   (i) CARACTERISTIQUES DE LA SEQUENCE:
      (A) LONGUEUR: 29 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovae
      (C) INDIVIDUEL ISOLE: oligonucleotide de reconstituion du gene defensine A
   (xi) DESCRIPTION DE LA SEQUENCE: SEQ ID NO: 7:
   AGCACAAGCG GAGTGGTTAA TACCGGTAC                                   29
(2) INFORMATION POUR LA SEQ ID NO: 8:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 8:
   AGAGGTAACA GAGGTGGCTA CTGTAACGGT AAGGGTGT                    38
(2) INFORMATION POUR LA SEQ ID NO: 9:
   (i) CARACTERISTIQUES DE LA SEQUENCE:
      (A) LONGUEUR: 37 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 9:
   AGTAGCCACC TCTGTTACCT CTCAACAAAC AGTGAGC                     37
(2) INFORMATION POUR LA SEQ ID NO: 10:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: Phormia terranovae
      (C) INDIVIDUEL ISOLE: oligonuclotide de reconstituion du gene defensine A
   (xi) DESCRIPTION DE LA SEQUENCE: SEQ ID NO: 10:
   TTGTGTTTGT AGAAACTAAG GATCCG                                  26
(2) INFORMATION POUR LA SEQ ID NO: 11:
   (i) CARACTERISTIQUES DE LA SEQUENCE:
      (A) LONGUEUR: 46 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovae
      (C) INDIVIDUEL ISOLE: oligonucleotide de reconstitution du gene defensine A
   (xi) DESCRIPTION DE LA SEQUENCE: SEQ ID NO: 11:
   AATTCGGATC CTTAGTTTCT ACAAACACAA ACACCCTTAC CGTTAC            46
(2) INFORMATION POUR LA SEQ ID NO: 12:
   (i) CARACTERISTIQUES DE LA SEQUENCE:
      (A) LONGUEUR: 37 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 12:
   TCGCAGCATC CTCCGCGCTA GCTGCTCCAG TCAACAC 37
(2) INFORMATION POUR LA SEQ ID NO: 13:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese
   (xi) DESCRIPTION DE LA SEQUENCE: SEQ ID NO: 13:
   ATGTTGCTCT CTTGTCAAAG CTTTGTAATC CTTGGC 36
(2) INFORMATION POUR LA SEQ ID NO: 14:
   (i) CARACTERISTIQUES DE LA SEQUENCE:
      (A) LONGUEUR: 88 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (B) SOUCHE: Phormia terranovae
      (C) INDIVIDUEL ISOLE: oligonucleotide de reconstitution de la sequence pro courte
   (xi) DESCRIPTION DE LA SEQUENCE: SEQ ID NO: 14:
   CTAGCTGCCA ACGATGCACA TTTTGTTGAT GGCGTTCAGG CTTTAAAAGA AATTGAACCC 60
   GAATTACATG GCCGTTACAA GAGAGCAA 88
(2) INFORMATION POUR LA SEQ ID NO: 15:
   (i) CARACTERISTIQUES DE LA SEQUENCE:
      (A) LONGUEUR: 88 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (B) SOUCHE: Phormia terranovae
      (C) INDIVIDUEL ISOLE: oligonucleotide de reconstitution de la sequence pro courte
   (xi) DESCRIPTION DE LA SEQUENCE: SEQ ID NO: 15:
   CATGTTGCTC TCTTGTAACG GCCATGTAAT TCGGGTTCAA TTTCTTTTAA AGCCTGAACG    60
   CCATCAACAA AATGTGCATC GTTGGCAG                                       88
(2) INFORMATION POUR LA SEQ ID NO: 16:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 16:
   TCCTCCGCGC TAGCTATTCC TGCTGATGCT GCCAACGATG CACAT                    45
(2) INFORMATION POUR LA SEQ ID NO: 17:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 17:
   GGCCGTTACA AGAGAATTAC GTATACAGAC GCAACATGTG ATTTA                    45
(2) INFORMATION POUR LA SEQ ID NO: 18:
   (i) CARACTERISTIQUES DE LA SEQUENCE:
      (A) LONGUEUR: 11 acides aminés
      (B) TYPE: acide aminé
      (D) CONFIGURATION: linéaire
   (ii) TYPE DE MOLECULE: peptide
   (iii) HYPOTHETIQUE: NON
   (v) TYPE DU FRAGMENT: N-terminal
   (vi) ORIGINE:
      (C) INDIVIDUEL ISOLE: sequencage N-term du materiel TGY47.1/pTG6823
   (xi) DESCRIPTION DE LA SEQUENCE: SEQ ID NO: 18:
   Ile Thr Tyr Thr Xaa Xaa Thr Glu Ser Gly Gln
   1               5                   10
(2) INFORMATION POUR LA SEQ ID NO: 19:
   (i) CARACTERISTIQUES DE LA SEQUENCE:
      (A) LONGUEUR: 11 acides aminés
      (B) TYPE: acide aminé
      (D) CONFIGURATION: linéaire
   (ii) TYPE DE MOLECULE: peptide
   (iii) HYPOTHETIQUE: NON
   (v) TYPE DU FRAGMENT: N-terminal
   (vi) ORIGINE:
      (C) INDIVIDUEL ISOLE: sequencage du materiel de la culture TGY47.1/pTG6823
   (xi) DESCRIPTION DE LA SEQUENCE: SEQ ID NO: 19:
   Tyr Xaa Xaa Ile Thr Tyr Thr Asp Xaa Thr Glu
   1               5                   10
(2) INFORMATION POUR LA SEQ ID NO: 20:
   (i) CARACTERISTIQUES DE LA SEQUENCE:
      (A) LONGUEUR: 15 acides aminés
      (B) TYPE: acide aminé
      (D) CONFIGURATION: linéaire
   (ii) TYPE DE MOLECULE: peptide
   (iii) HYPOTHETIQUE: NON
   (v) TYPE DU FRAGMENT: N-terminal
   (vi) ORIGINE:
      (C) INDIVIDUEL ISOLE: sequencage du materiel de la culture TGY47.1/pTG6824
   (xi) DESCRIPTION DE LA SEQUENCE: SEQ ID NO: 20:
   Ile Thr Tyr Thr Asp Xaa Thr Glu Ser Gly Gln Asn Leu Xaa Leu
   1               5                   10                  15
(2) INFORMATION POUR LA SEQ ID NO: 21:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 21:
   GATGCTGCCA ACGATACACA TTTTGTTGAT GGC                          33
(2) INFORMATION POUR LA SEQ ID NO: 22:
   (i) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 22:
   GTTACAAGAG AATTACGTGC TCCATGTTAC CTATCTCAG                    39
(2) INFORMATION POUR LA SEQ ID NO: 23:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (C) INDIVIDUEL ISOLE: oligonucleotide de mutagenese
   (xi) DESCRIPTION DE LA SEQUENCE: SEQ ID NO: 23:
   CATGGCCGTT ACAAGAGAGC TCCATGTTAC CTATCT                       36
(2) INFORMATION POUR LA SEQ ID NO: 24:
   (i) CARACTERISTIQUES DE LA SEQUENCE:
      (A) LONGUEUR: 13 acides aminés
      (B) TYPE: acide aminé.
      (D) CONFIGURATION: linéaire
   (ii) TYPE DE MOLECULE: peptide
   (iii) HYPOTHETIQUE: NON
   (v) TYPE DU FRAGMENT: N-terminal
   (vi) ORIGINE:
      (C) INDIVIDUEL ISOLE: sequencage du materiel de la culture TGY47.1/pTG8840
   (xi) DESCRIPTION DE LA SEQUENCE: SEQ ID NO: 24:
   Ala Pro Xaa Tyr Leu Ser Gln Arg Leu Met Leu Asp Ala
   1               5                   10
(2) INFORMATION POUR LA SEQ ID NO: 25:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4864
      (C) INDIVIDUEL ISOLE: oligonucleotide de mutagenese (pro def A Pro2 -> Cys)
   (vii) SOURCE IMMEDIATE:
      (B) CLONE: M13TG9836
   (xi) DESCRIPTION DE LA SEQUENCE: SEQ ID NO: 25:
   TCCGCGCTAG CTATTTGTGC TGATGCTGCC AA                                32
(2) INFORMATION POUR LA SEQ ID NO: 26:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4865
      (C) INDIVIDUEL ISOLE: oligonucleotide de mutagenese (sequ pro def A Ala6 -> Cys)
   (vii) SOURCE IMMEDIATE:
      (B) CLONE: M13TG9838
   (xi) DESCRIPTION DE LA SEQUENCE: SEQ ID NO: 26:
   ATTCCTGCTG ATGCTCTTAA CGATGCACAT TT                                32
(2) INFORMATION POUR LA SEQ ID NO: 27:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4866
      (C) INDIVIDUEL ISOLE: oligonucleotide de mutagenese (sequence pro def A Ala9->Cys)
   (vii) SOURCE IMMEDIATE:
      (B) CLONE: M13TG9839
   (xi) DESCRIPTION DE LA SEQUENCE: SEQ ID NO: 27:
   GATGCTGCCA ACGATTGTCA TTTTGTTGAT GGC 33
(2) INFORMATION POUR LA SEQ ID NO: 28:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4867
      (C) INDIVIDUEL ISOLE: oligonucleotide de mutagenese (sequ pro def A Ala17 -> Cys)
   (vii) SOURCE IMMEDIATE:
      (B) CLONE: M13TG9840
   (xi) DESCRIPTION DE LA SEQUENCE: SEQ ID NO: 28:
   GTTGATGGCG TTCAGTGTTT AAAAGAAATT GAA 33
(2) INFORMATION POUR LA SEQ ID NO: 29:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG3051
      (C) INDIVIDUEL ISOLE: oligonucleotide de mutagenese
   (xi) DESCRIPTION DE LA SEQUENCE: SEQ ID NO: 29:
   TCCTCCGCGC TAGCTATTCC TGCTGATGCT GCCAACGATG CACAT 45
(2) INFORMATION POUR LA SEQ ID NO: 30:
   (i) CARACTERISTIQUES DE LA SEQUENCE:
      (A) LONGUEUR: 75 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG3070
      (C) INDIVIDUEL ISOLE: oligonucleotide
   (xi) DESCRIPTION DE LA SEQUENCE: SEQ ID NO: 30:
   TCGAGTAAAA GAATGAAATT CTTCATGGTA TTCGTTGTTA CTTTCTGCTT AGCAGTTTGT 60
   TTTGTCTCCC AATCG 75
(2) INFORMATION POUR LA SEQ ID NO: 31:
   (i) CARACTERISTIQUES DE LA SEQUENCE:
      (A) LONGUEUR: 75 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG3071
      (C) INDIVIDUEL ISOLE: oligonucleotide
   (xi) DESCRIPTION DE LA SEQUENCE: SEQ ID NO: 31:
   CTAGCGATTG GGAGACAAAA CAAACTGCTA AGCAGAAAGT AACAACGAAT ACCATGAAGA        60
   ATTTCATTCT TTTAC                                                         75
(2) INFORMATION POUR LA SEQ ID NO: 32:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4442
      (C) INDIVIDUEL ISOLE: amorce PCR d'un fragment "prom MFalphal-sequ pre BGL2"
   (xi) DESCRIPTION DE LA SEQUENCE: SEQ ID NO: 32:
   CTCGATATCG CATGCTATTG ATAAGATTTA AAGGTA                                  36
(2) INFORMATION POUR LA SEQ ID NO: 33:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG4443
      (C) INDIVIDUEL ISOLE: amorce PCR pour un fragment "prom MFalphal-sequ pre BGL2"
   (xi) DESCRIPTION DE LA SEQUENCE: SEQ ID NO: 33:
   TGTGCATCGT TGGCAGCATC AGCAGGAATA GCTGAAAC                          38
(2) INFORMATION POUR LA SEQ ID NO: 34:
   (i) CARACTERISTIQUES DE LA SEQUENCE:
      (A) LONGUEUR: 59 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4444
      (B) SOUCHE: amorce PCR pour isoler sequence pro def A (site EagI)
   (xi) DESCRIPTION DE LA SEQUENCE: SEQ ID NO: 34:
   CTATTTTTCA CAGCCTCCCA AGTTTCAGCT ATTCCTGCTG ATGCTGCCAA CGATGCACA   59
(2) INFORMATION POUR LA SEQ ID NO: 35:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG4445
      (B) SOUCHE: amorce PCR pour isoler sequence pro def A (site EagI)
   (xi) DESCRIPTION DE LA SEQUENCE: SEQ ID NO: 35:
   AAGCTAAGTG GTACCTCTCT TGTAACGGCC GTGTAATTCG GGTTC 45
(2) INFORMATION POUR LA SEQ ID NO: 36:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG4637
      (C) INDIVIDUEL ISOLE: oligonucleotide de mutagenese (correction mutation prom MF1)
   (vii) SOURCE IMMEDIATE:
      (B) CLONE: M13TG9800
   (xi) DESCRIPTION DE LA SEQUENCE: SEQ ID NO: 36:
   TAAACGATTA AAAGAATGCG T 21
(2) INFORMATION POUR LA SEQ ID NO: 37:
   (i) CARACTERISTIQUES DE LA SEQUENCE:
      (A) LONGUEUR: 42 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG5074
      (C) INDIVIDUEL ISOLE: amorce PCR
   (xi) DESCRIPTION DE LA SEQUENCE: SEQ ID NO: 37:
   GCATGATCCT CGGCCGTTAC AAGAGAATTA CGTATACAGA CT 42
(2) INFORMATION POUR LA SEQ ID NO: 38:
   (i) CARACTERISTIQUES DE LA SEQUENCE:
      (A) LONGUEUR: 43 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG5075
      (C) INDIVIDUEL ISOLE: amorce PCR
   (xi) DESCRIPTION DE LA SEQUENCE: SEQ ID NO: 38:
   GATGCTGTGA GTCGACCATT TTTCATTGTA AATATTCTTC TGG                43
(2) INFORMATION POUR LA SEQ ID NO: 39:
   (i) CARACTERISTIQUES DE LA SEQUENCE:
      (A) LONGUEUR: 22 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG5136
      (C) INDIVIDUEL ISOLE: adaptateur
   (xi) DESCRIPTION DE LA SEQUENCE: SEQ ID NO: 39:
   GGCCCATACA AGAGAATTAC GT                                       22
(2) INFORMATION POUR LA SEQ ID NO: 40:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG5137
      (C) INDIVIDUEL ISOLE: adaptateur
   (xi) DESCRIPTION DE LA SEQUENCE: SEQ ID NO: 40:
   ATACGTAATT CTCTTGTATG 20
(2) INFORMATION POUR LA SEQ ID NO: 41:
   (i) CARACTERISTIQUES DE LA SEQUENCE:
      (A) LONGUEUR: 45 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: OTG5770
      (C) INDIVIDUEL ISOLE: amorce de PCR
   (xi) DESCRIPTION DE LA SEQUENCE: SEQ ID NO: 41:
   TACGTCGGCC GTTACAAGAG ACTGAAATGT GTGAATTTAC AAGCG 45
(2) INFORMATION POUR LA SEQ ID NO: 42:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: OTG5771
      (C) INDIVIDUEL ISOLE: amorce de PCR
   (xi) DESCRIPTION DE LA SEQUENCE: SEQ ID NO: 42:
   AGTTTGTCGA CTCAAGCATT GCACATGTTT CCT                                   33
(2) INFORMATION POUR LA SEQ ID NO: 43:
   (i) CARACTERISTIQUES DE LA SEQUENCE:
      (A) LONGUEUR: 66 acides aminés
      (B) TYPE: acide aminé
      (D) CONFIGURATION: linéaire
   (ii) TYPE DE MOLECULE: protéine
   (iii) HYPOTHETIQUE: NON
   (vi) ORIGINE:
      (A) ORGANISME: Xenoxine
      (B) SOUCHE: Xenopus laevis
   (ix) CARACTERISTIQUE ADDITIONELLE:
      (A) NOM/CLE: Protein
      (B) EMPLACEMENT: 1..61
   (xi) DESCRIPTION DE LA SEQUENCE: SEQ ID NO: 43:
   Leu Lys Cys Val Asn Leu Gln Ala Asn Gly Ile Lys Met Thr Gln Glu
   1               5                   10                  15
   Cys Ala Lys Glu Asp Thr Lys Cys Leu Thr Leu Arg Ser Leu Lys Lys
               20                  25                  30
   Thr Leu Lys Phe Cys Ala Ser Gly Arg Thr Cys Thr Thr Met Lys Ile
           35                  40                  45
   Met Ser Leu Pro Gly Glu Gln Ile Thr Cys Cys Glu Gly Asn Met Cys
       50                  55                  60
   Asn Ala
   65
(2) INFORMATION POUR LA SEQ ID NO: 44:
   (i) CARACTERISTIQUES DE LA SEQUENCE:
      (A) LONGUEUR: 15 acides aminés
      (B) TYPE: acide aminé
      (D) CONFIGURATION: linéaire
   (ii) TYPE DE MOLECULE: peptide
   (iii) HYPOTHETIQUE: NON
   (v) TYPE DU FRAGMENT: N-terminal
   (vi) ORIGINE:
      (C) INDIVIDUEL ISOLE: sequencage du materiel de la culture TGY73.4/pTG8709
   (xi) DESCRIPTION DE LA SEQUENCE: SEQ ID NO: 44:
   Leu Lys Xaa Val Asn Leu Gln Ala Asn Gly Ile Lys Met Xaa Gln
   1               5                   10                  15